# FASCICULE DE BREVET EUROPEEN

(11) **EP 3 049 168 B1**
(45) Date de publication et mention de la délivrance du brevet: **31.08.2022**
(21) Numéro de dépôt: 14786981.2
(22) Date de dépôt: 26.08.2014
(51) Int. Cl.: C07C 67/08, C07C 67/58, B01D 11/04

(54) **PROCEDE D'EXTRACTION LIQUIDE-LIQUIDE POUR LA PRODUCTION D'ESTERS ACRYLIQUES**
FLÜSSIG-FLÜSSIG-EXTRAKTIONSVERFAHREN ZUR HERSTELLUNG VON ACRYLESTERN
LIQUID-LIQUID EXTRACTION METHOD FOR THE PRODUCTION OF ACRYLIC ESTERS

(30) Priorité: 23.09.2013 FR 1359110
(43) Date de publication de la demande: 03.08.2016
(73) Titulaire: Arkema France, 92700 Colombes (FR)
(72) Inventeur: TRETJAK, Serge, 57520 Roulhing (FR); DENIS, Stephane, 57660 Leyviller (FR)
(74) Mandataire: Arkema Patent
(86) Numéro de dépôt international: PCT/FR2014/052124
(87) Numéro de publication internationale: WO 2015/040298

(56) Documents cités:
- EP-A1- 1 721 886
- FR-A1- 2 502 638
- FR-A1- 2 735 460
- JP-A- 2012 140 355
- US-A- 3 821 286
- US-A1- 2005 107 629

## Description

### DOMAINE DE L'INVENTION

La présente invention concerne un procédé d'extraction liquide - liquide, ainsi que l'utilisation de ce procédé dans le cadre de la production d'esters acryliques, et notamment d'acrylate de méthyle et d'acrylate d'éthyle.

### ARRIERE-PLAN TECHNIQUE

On connaît des procédés de fabrication d'esters acryliques, et notamment d'acrylate de méthyle et d'acrylate d'éthyle, par estérification directe de l'acide acrylique par l'alcool correspondant, catalysée par exemple par l'acide sulfurique. Dans de tels procédés, des quantités importantes d'alcool non réagi sont collectées à la sortie du réacteur.

Pour des raisons environnementales et économiques, il est indispensable de récupérer l'alcool non réagi, afin généralement de le recycler au réacteur.

A cet effet, il est connu de distiller le flux de produits, ou encore de le laver au moyen d'un flux aqueux.

Les documents FR 2509294, US 5,435,892 et FR 2884514 fournissent des exemples de tels procédés de production d'esters acryliques.

Dans le document EP 1721886, le mélange brut d'ester acrylique est soumis à différents traitements visant à séparer le catalyseur, à concentrer et purifier le produit recherché. Ces traitements peuvent être effectués par lavage, neutralisation et/ou extraction. Les exemples d'appareillage cités sont les mélangeurs/décanteurs, les colonnes d'extraction ou les réservoirs agités. Les colonnes d'extraction sont de type à garnissage, à plateaux ou à disque rotatif. Le procédé de production d'acrylate de méthyle est illustré à l'aide d'un traitement associant une extraction du mélange réactionnel dans une colonne à garnissage avec une filtration. Cependant, la teneur en alcool résiduel dans la phase organique extraite reste de l'ordre de 0,1 à 0,2% massique.

Il existe encore un besoin de parvenir à récupérer l'alcool en sortie du réacteur de manière plus efficace.

### RESUME DE L'INVENTION

L'invention concerne en premier lieu un procédé d'extraction liquide - liquide, comprenant :
- la fourniture d'un flux liquide principal d'entrée, contenant au moins un composé d'intérêt et un composé excédentaire ;
- la fourniture d'un flux liquide de lavage ;
- l'extraction du composé excédentaire du flux liquide principal d'entrée par contact avec le flux liquide de lavage, permettant de collecter un flux liquide principal de sortie, appauvri en composé excédentaire par rapport au flux liquide principal d'entrée ;
dans lequel :
- le flux liquide principal d'entrée et le flux liquide de lavage présentent une différence de masse volumique inférieure ou égale à 50 kg/m³ ainsi qu'une tension interfaciale inférieure ou égale à 3 dyn/cm ; et
- l'étape d'extraction est effectuée dans un contacteur à garnissage avec un rapport massique en entrée flux liquide de lavage / flux liquide principal allant de 0,3 à 0,5.
- le flux liquide principal d'entrée est un flux organique et le flux liquide de lavage est un flux aqueux ;
- le composé d'intérêt est un ester acrylique, de manière plus particulièrement préféré l'acrylate de méthyle et/ou l'acrylate d'éthyle ; et le composé excédentaire est un alcool, de préférence le méthanol et/ou l'éthanol.

Selon un mode de réalisation le flux liquide principal d'entrée contient de 0,5 à 30 % de composé excédentaire, de préférence de 1 à 20 % et plus particulièrement de 2 à 10 % ; et/ou le flux liquide principal de sortie contient moins de 2000 ppm de composé excédentaire, de préférence moins de 1000 ppm, et plus particulièrement moins de 750 ppm voire moins de 500 ppm.

Selon un mode de réalisation le contacteur à garnissage comporte des éléments de garnissage présentant un diamètre supérieur ou égal au diamètre critique, exprimé en m, résultant de la relation d_{c}=2,42×(S*gc_{/}Δρ*g)^{0,5}, S représentant la tension interfaciale entre le flux liquide principal d'entrée et le flux liquide de lavage, exprimée en N/m, et Δρ représentant la différence de masse volumique entre le flux liquide principal d'entrée et le flux liquide de lavage, exprimée en kg/m³, gc étant le facteur de conversion de la constante de pesanteur (kg.m/N.s²) et g la constante gravitationnelle (9,83 m/s²).

La surface spécifique du garnissage (en vrac ou structuré) du contacteur est supérieure ou égale à 200 m²/m³.

L'invention a également pour objet un procédé de production d'ester acrylique comprenant :
- l'alimentation d'un réacteur en acide acrylique, en catalyseur et en alcool ;
- le soutirage d'un flux d'ester acrylique en sortie du réacteur ;
- l'extraction liquide - liquide du flux d'ester acrylique par un flux aqueux, permettant de collecter un flux d'ester acrylique purifié, l'extraction liquide - liquide étant effectuée selon le procédé décrit ci-dessus, dans lequel le flux liquide principal d'entrée est le flux d'ester acrylique, le flux liquide de lavage est le flux aqueux, le flux liquide principal de sortie est le flux d'ester acrylique purifié, le composé d'intérêt est l'ester acrylique, et le composé excédentaire est l'alcool.

Selon un mode de réalisation le flux d'ester acrylique purifié est soumis en outre à une ou plusieurs étapes de distillation afin d'éliminer des composés organiques supplémentaires.

Selon un mode de réalisation le procédé comprend une étape de décantation en sortie du réacteur, permettant de collecter une phase aqueuse en plus du flux d'ester acrylique, la phase aqueuse étant distillée pour récupérer d'une part une fraction riche en alcool qui est recyclée vers le réacteur, et d'autre part une fraction riche en eau qui est utilisée en tant que flux liquide de lavage dans l'étape d'extraction liquide-liquide.

Selon un mode de réalisation, le procédé comprend la collecte d'un flux aqueux enrichi en alcool à l'issue de l'étape d'extraction liquide-liquide et la combinaison de celui-ci avec la phase aqueuse issue du réacteur.

L' installation de production d'ester acrylique comprend :
- un réacteur alimenté par une conduite d'amenée d'acide acrylique, une conduite d'amenée d'alcool et une conduite d'amenée de catalyseur ;
- une conduite de collecte d'ester acrylique en sortie du réacteur ;
- une unité d'extraction liquide-liquide, comprenant un contacteur à garnissage, alimentée par la conduite de collecte d'ester acrylique ainsi que par une conduite d'alimentation en flux liquide de lavage ;
- une conduite de collecte d'ester acrylique purifié en sortie de l'unité d'extraction liquide-liquide.

L'unité d'extraction comprend un contacteur à garnissage comportant des éléments de garnissage présentant un diamètre supérieur ou égal au diamètre critique, exprimé en m, résultant de la relation d_{c}=2,42×(S*gc_{/}Δρ*g)^{0,5}, S représentant la tension interfaciale entre le flux d'ester acrylique et le flux liquide de lavage, exprimée en N/m, et Δρ représentant la différence de masse volumique entre le flux liquide principal d'entrée et le flux liquide de lavage, exprimée en kg/m³, gc étant le facteur de conversion de la constante de pesanteur (kg.m/N.s²) et g la constante gravitationnelle (9,83 m/s²).

Selon un mode de réalisation, la conduite de collecte d'ester acrylique purifié alimente une ou plusieurs unités de distillation.

Selon un mode de réalisation, l'installation comprend:
- une conduite de collecte de phase aqueuse en sortie du réacteur ;
- une unité de distillation hydroalcoolique, alimentée par la conduite de collecte de phase aqueuse et optionnellement en outre par une conduite de collecte de flux aqueux enrichi en alcool issue de l'unité d'extraction liquide-liquide, la conduite d'alimentation en flux liquide de lavage étant connectée en sortie de l'unité de distillation hydroalcoolique ;
- une conduite de collecte de fraction riche en alcool, connectée en sortie de l'unité de distillation hydroalcoolique et alimentant le réacteur.

La conduite d'amenée d'alcool est une conduite d'amenée de méthanol ou une conduite d'amenée d'éthanol.

La présente invention permet de surmonter les inconvénients de l'état de la technique. Elle fournit plus particulièrement un moyen de récupération de l'alcool non réagi en sortie d'un réacteur de production d'ester acrylique plus efficace.

Cela est accompli grâce à une extraction liquide-liquide améliorée, reposant sur l'utilisation d'un contacteur à garnissage.

Les inventeurs ont en effet découvert que, lorsque les flux liquides en contact dans une unité d'extraction liquide-liquide présentent une faible différence de masse volumique et une faible tension interfaciale, l'utilisation d'un tel contacteur à garnissage est plus avantageuse que celle d'un contacteur classique à agitation mécanique, en ce qu'elle offre une productivité supérieure (à performance d'extraction similaire) - étant précisé que d'autres types de contacteurs, tels que les contacteurs centrifuges par exemple, présentent l'inconvénient d'être plus complexes et donc plus onéreux.

Des flux liquides ayant les propriétés susmentionnées présentent une difficulté de traitement particulière, car la séparation a tendance à être peu efficace, du fait d'une taille de gouttelettes relativement élevée (supérieure à 0,6 mm). Il a notamment été constaté une tendance à l'engorgement dans les contacteurs à agitation mécanique, sauf à utiliser des débits faibles.

Ainsi, l'invention permet d'utiliser un contacteur de plus petite taille et moins onéreux afin d'atteindre la productivité recherchée et les spécifications souhaitées en taux d'alcool résiduel dans le flux de produits.

### BREVE DESCRIPTION DES FIGURES

La **figure 1** représente de manière schématique une installation de production d'ester acrylique selon un mode de réalisation de l'invention.
La **figure 2** illustre un exemple de contacteur à garnissage utilisable dans le cadre de l'invention. Le dessin de gauche est une vue en coupe du contacteur selon un plan vertical. Les dessins de droite sont des vues de détails de l'appareil (coupe selon un plan vertical en haut et selon un plan horizontal en bas).

### DESCRIPTION DE MODES DE REALISATION DE L'INVENTION

L'invention est maintenant décrite plus en détail et de façon non limitative dans la description qui suit.

Les pourcentages indiqués sont des pourcentages massiques sauf mention contraire.

### Production d'ester acrylique

Par souci de simplicité de l'exposé, le procédé d'extraction liquide-liquide de l'invention est décrit en référence à un procédé de production d'ester acrylique.

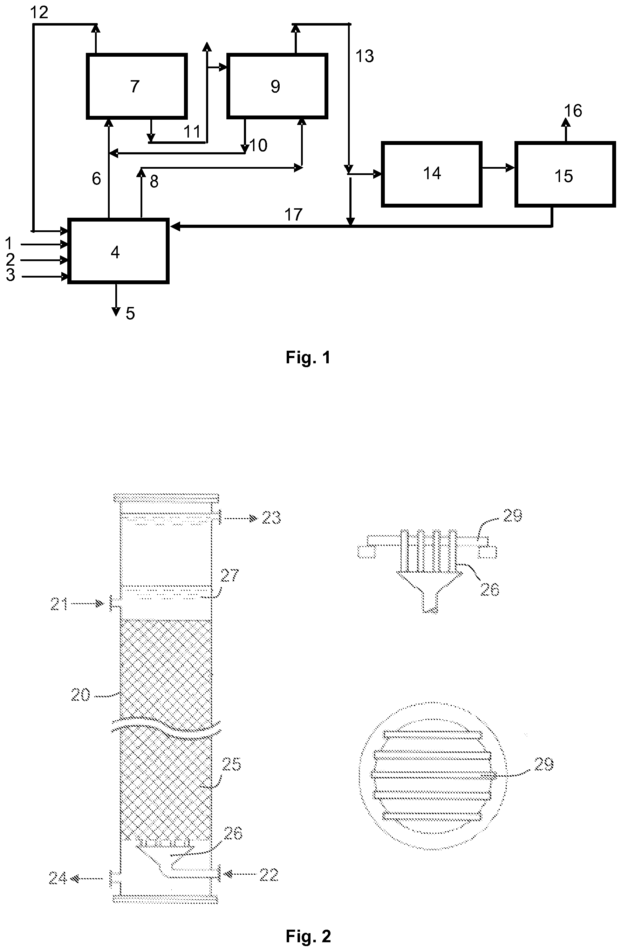

En faisant référence à la **figure 1**, une installation de production d'ester acrylique selon l'invention comporte un réacteur 4. Le réacteur 4 est alimenté par une conduite d'amenée d'acide acrylique 2, une conduite d'amenée d'alcool 3 et une conduite d'amenée de catalyseur 1.

Selon un mode de réalisation, l'alcool est du méthanol. Selon un autre mode de réalisation, l'alcool est de l'éthanol. Un mélange de méthanol et d'éthanol est également possible. A titre de catalyseur on peut utiliser par exemple de l'acide sulfurique, ou un acide organique sulfonique, tel que l'acide méthane sulfonique, l'acide para-toluène sulfonique, l'acide benzène sulfonique, l'acide dodécyl sulfonique, ou leurs mélanges.

En sortie du réacteur 4, les produits lourds sont éliminés *via* une collecte de purge de produits lourds 5.

Au moyen d'un décanteur (non représenté) associé au réacteur 4, on récupère en sortie du réacteur 4 : d'une part un flux d'ester acrylique *via* une conduite de collecte d'ester acrylique 8 ; et d'autre part une phase aqueuse (contenant également une partie de l'alcool non réagi) *via* une conduite de collecte de phase aqueuse 6.

Le flux d'ester acrylique comprend de l'ester acrylique à titre de « *composé d'intérêt* » (au sens de l'invention), ainsi que des sous-produits, contaminants et réactifs non réagis, et notamment le composé alcool mentionné ci-dessus, qui constitue le « *composé excédentaire* » au sens de l'invention.

L'ester acrylique (composé d'intérêt) est de préférence de l'acrylate de méthyle et/ou de l'acrylate d'éthyle. L'alcool non réagi (composé excédentaire) est de préférence du méthanol et/ou de l'éthanol.

Le flux d'ester acrylique peut comprendre par exemple de 50 à 98 % d'ester acrylique, de préférence de 70 à 97 %, et plus particulièrement de 80 à 95 %. Ce même flux d'ester acrylique peut comprendre par exemple de 0,5 à 30 % d'alcool non réagi, de préférence de 1 à 20 %, plus particulièrement de 2 à 10%.

Le flux d'ester acrylique décrit ci-dessus constitue le « *flux liquide principal d'entrée* » au sens de l'invention.

La conduite de collecte de phase aqueuse 6 alimente une unité de distillation hydroalcoolique 7. Cette unité de distillation hydroalcoolique 7 permet de récupérer l'alcool non réagi, et de le recycler au réacteur 4 *via* une conduite de collecte de fraction riche en alcool 12. Le reste de la phase aqueuse (fraction riche en eau) est récupéré en pied de l'unité de distillation hydroalcoolique 7 afin d'être utilisé en tant que flux liquide de lavage (au sens de l'invention), *via* une ligne appelée conduite d'alimentation en flux liquide de lavage 11. Il faut noter que la totalité de ce flux n'est pas nécessairement utilisée en tant que flux liquide de lavage, une partie pouvant être éliminée, ainsi que cela est représenté sur le schéma par la flèche verticale.

La conduite d'alimentation en flux liquide de lavage 11 et la conduite de collecte d'ester acrylique 8 alimentent toutes deux une unité d'extraction liquide-liquide 9, dans laquelle une extraction liquide-liquide est effectuée entre les deux flux, permettant de transférer le composé excédentaire (alcool non réagi) du flux d'ester acrylique vers le flux liquide de lavage.

L'unité d'extraction liquide-liquide est mise en fonctionnement avec un rapport massique en entrée flux liquide de lavage / flux liquide principal allant de 0,3 à 0,5. En effet, il a été montré qu'en-dessous d'un rapport massique de 0,3, le composé excédentaire restait encore présent à une teneur importante dans le flux liquide principal de sortie. En particulier, pour un rapport massique de 0,3 dans un procédé de production d"ester acrylique l'alcool résiduel dans le flux de sortie d'ester acrylique purifié est supérieur à 3000 ppm, ce qui n'est pas compatible avec les utilisations finales de ce monomère. Par ailleurs, l'utilisation d'un rapport supérieur à 0,5 implique la mise en œuvre d'un grand volume de liquide de lavage et l'utilisation d'une colonne d'extraction plus grande pour obtenir la même efficacité d'extraction, ce qui n'est pas économiquement avantageux.

Cette unité d'extraction liquide-liquide 9 est décrite plus en détail ci-dessous.

En sortie de l'unité d'extraction liquide-liquide 9 sont connectées d'une part une conduite de collecte d'ester acrylique purifié 13 (afin de récupérer le flux d'ester acrylique appauvri en alcool) et d'autre part une conduite de collecte de flux aqueux enrichi en alcool 10 (afin de récupérer le flux liquide de lavage enrichi en alcool).

La conduite de collecte de flux aqueux enrichi en alcool 10 retourne vers l'unité de distillation hydroalcoolique 7 : le flux (de lavage) enrichi en alcool est ainsi combiné avec la phase aqueuse issue du réacteur, permettant de récupérer et de recycler l'alcool qu'il contient.

La conduite de collecte d'ester acrylique purifié 13 alimente des unités de distillation successives 14, 15, qui permettent d'éliminer d'autres composés indésirables et de récupérer l'ester acrylique sous sa forme finale *via* une ligne de collecte de produit final 16. Une ou plusieurs lignes de recyclage 17 peuvent être prévues pour retourner une partie du flux au réacteur 4, alimentées par la conduite de collecte d'ester acrylique purifié 13 et/ou les unités de distillation successives 14, 15.

La teneur résiduelle en alcool dans le flux récupéré dans la conduite de d'ester acrylique purifié 13 est de préférence inférieure ou égale à 2000 ppm ou à 1000 ppm, par exemple inférieure ou égale à 750 ou à 500 ppm.

### Unité d'extraction liquide-liquide

L'invention prévoit d'utiliser un contacteur à garnissage pour l'unité d'extraction liquide-liquide 9 ci-dessus. Selon un mode de réalisation l'unité d'extraction liquide-liquide 9 consiste en un contacteur à garnissage unique, ou en une pluralité de contacteurs à garnissage disposés en série ou en parallèle. Selon un mode de réalisation alternatif, l'unité d'extraction liquide-liquide 9 comporte en complément un contacteur d'un autre type, par exemple un contacteur à agitation mécanique. Selon encore un autre mode de réalisation, des moyens complémentaires d'élimination du composé excédentaire sont associés à l'unité d'extraction liquide-liquide (par exemple une unité de distillation).

Par « *contacteur à garnissage* » on entend un appareil comprenant une enceinte dans laquelle deux liquides sont mis en contact, l'enceinte comportant un garnissage. Par « *garnissage* » on entend une structure solide propre à accroître la surface de contact entre les deux liquides.

De préférence, le contacteur à garnissage est un contacteur statique, c'est-à-dire dépourvu de moyens d'agitation mécanique (tels que pales, turbines, etc.) dans l'enceinte susmentionnée.

En faisant référence à la **figure 2****,** un exemple de contacteur à garnissage utilisable dans le cadre de l'invention comporte une colonne 20 formant une enceinte. Une conduite d'alimentation en phase lourde 21 et une conduite d'alimentation en phase légère 22 sont connectées en entrée de la colonne 20, respectivement en tête et en pied de celle-ci. Une conduite de collecte de phase légère 23 et une conduite de collecte de phase lourde 24 sont connectées en sortie de la colonne 20, respectivement en tête et en pied de celle-ci.

La colonne 20 contient un garnissage 25 qui repose sur un plateau 29 sous forme de grille. Un système de distribution 26 est prévu au niveau de l'arrivée de la conduite d'alimentation en phase légère 22. Ce système de distribution 26 comporte par exemple un ensemble de buses, afin de permettre de générer des gouttes de phase légère dans la phase lourde. Il est disposé en dessous du plateau 29 et peut traverser celui-ci.

Au-dessus du garnissage 25, une zone de décantation 27 est ménagée, permettant la séparation de la phase lourde et de la phase légère. Les débits sont ajustés de sorte que l'interface entre les phases soit située entre la conduite d'alimentation en phase lourde 21 et la conduite de collecte de phase légère 23 (placée au-dessus de la précédente).

Dans le cadre de la production d'esters acryliques décrite ci-dessus, la phase légère est le flux liquide principal (flux d'ester acrylique), et la phase lourde est le flux liquide de lavage (de nature aqueuse).

La phase lourde est la phase continue, dans laquelle sont dispersées des gouttes de phase légère au niveau du garnissage 25.

La température d'extraction est de préférence de 20 à 50°C. Le rapport massique en entrée phase lourde / phase légère est de préférence de 0,3 à 0,5.

Le garnissage peut être un garnissage en vrac ou un garnissage structuré ou éventuellement une combinaison des deux, de préférence un garnissage en vrac. Un garnissage en vrac est composé d'objets poreux entassés, les éléments de garnissage, qui peuvent par exemple avoir une forme externe essentiellement cylindrique. Un garnissage structuré est composé d'une structure tridimensionnelle poreuse unique, ou sous forme de blocs arrangés les uns au-dessus des autres et/ou les uns à côté des autres.

Le garnissage peut être fabriqué avec un matériau céramique, ou métallique ou en verre, voire éventuellement en matière plastique. Le matériau préféré est de l'acier inoxydable.

Le diamètre externe des éléments de garnissage (dans le cas d'un garnissage en vrac) est de préférence de 5 à 50 mm. De manière générale, ce diamètre externe est choisi supérieur ou égal au diamètre critique d_{c} présenté dans Perry's Chemical Engineer's Handbook 7th edition, authors R.H.Perry and D.W.Green, chapter 15- Liquid-Liquid extraction opérations and equipment). Le diamètre d_{c}, exprimé en m, résulte de la relation d_{c}=2,42×(S*gc_{/}Δρ*g)^{0,5}, S représentant la tension interfaciale entre le flux liquide principal d'entrée et le flux liquide de lavage, exprimée en N/m, et Δρ représentant la différence de masse volumique entre le flux liquide principal d'entrée (flux d'ester acrylique) et le flux liquide de lavage, exprimée en kg/m³, gc étant le facteur de conversion de la constante de pesanteur (kg.m/N.s²) et g la constante gravitationnelle (9,83 m/s²).

Dans ces conditions, la taille des gouttes formées et par conséquent l'aire d'échange sont pratiquement indépendants du choix du garnissage.

De préférence, le diamètre des éléments de garnissage est inférieur au dixième du diamètre de la colonne d'extraction pour réduire l'encrassement de la colonne.

Les éléments de garnissage peuvent être par exemple des anneaux de Raschig, des anneaux de Pall, des anneaux de Saddle, des selles de Berl ou des selles Intalox. Alternativement, des billes pourraient également être utilisées. La surface spécifique des éléments de garnissage est supérieure ou égale à 200 m²/m³.

Lorsqu'un garnissage structuré est utilisé, on le choisit avantageusement de telle sorte qu'il présente un rapport surface / volume d'au moins 200 m²/m³, par exemple d'au moins 250 m²/m³, ou d'au moins 500 m²/m³. Des garnissages structurés possibles disponibles dans le commerce sont le garnissage BX de Sulzer à 250 m²/m³ et le Mellapack 750Y, également de Sulzer, à 750 m²/m³.

### EXEMPLE

L'exemple suivant illustre l'invention sans la limiter.

On compare dans cet exemple l'efficacité d'un contacteur à garnissage et d'un contacteur à agitation mécanique pour effectuer l'extraction liquide-liquide du méthanol contenu dans un flux d'acrylate de méthyle.

A titre de contacteur à agitation mécanique, on utilise une colonne de type Kuhni 150/30 G ayant une hauteur de 3,9 m, avec cinq sections de 420 mm comprenant chacune six agitateurs. Le diamètre de la colonne est de 150 mm, et la colonne comporte une zone de décantation en tête et une zone de tranquillisation en pied.

A titre de contacteur à garnissage, on utilise une colonne de 180 mm de diamètre, avec une hauteur active de 4 à 5 m. Le garnissage est constitué d'anneaux de Pall d'un diamètre de 16 mm, et de surface spécifique 205 m²/m³. Le décanteur en tête présente un diamètre de 150 mm, et une zone de tranquillisation est prévue en pied.

Le flux à traiter (flux organique) est prélevé dans une installation de production d'acrylate de méthyle. Il contient 91,09 % d'acrylate de méthyle et 5,03 % de méthanol. Les principaux autres composés détectés dans le flux sont l'acétate de méthyle (1,94 %) et l'acide acrylique (0,21 %).

Le flux de lavage est une phase aqueuse contenant 0,1 % de méthanol.

La différence de densité entre le produit de tête de colonne et celui du pied est dans ce cas de 30 kg/m³. Les densités des produits sont mesurées à 20°C à l'aide d'une fiole jaugée et confirmées avec le logiciel de simulation ASPEN. La tension interfaciale est de 3 dyn/cm. Elle est mesurée à 20°C grâce à un tensiomètre à goutte IT concept équipé d'un ordinateur, d'un pousse seringue de type EXMIRE, d'une caméra et d'une cuve en verre, le tout couplé à un bain thermostaté HAAKE.

Dans le tableau 1 ci-dessous sont rassemblés les résultats obtenus avec le contacteur à agitation mécanique, dans différentes conditions opérationnelles. Dans ce tableau, PO désigne la phase organique et PA désigne la phase aqueuse.

**Tableau 1 - résultats obtenus avec le contacteur à agitation mécanique**

| Débit PO en entrée (kg/h) | Débit PA en entrée (kg/h) | Débit PO en sortie (kg/h) | Débit PA en sortie (kg/h) | Rapport PA/PO à l'entrée | Productivité (m³/m²/h) | Teneur résiduelle méthanol dans PO en sortie (ppm) | Vitesse d'agitation (rpm) |
|---|---|---|---|---|---|---|---|
| 144,7 | 58,1 | 136,0 | 70,8 | 0,401 | 11,9 | 139 | 130 |
| 144,7 | 58,1 | 136,0 | 70,8 | 0,401 | 11,9 | 139 | 110 |
| 178,0 | 74,2 | 161,0 | 93,5 | 0,417 | 14,8 | 134 | 90 |
| 176,5 | 74,6 | 133,1 | 96,0 | 0,423 | 14,8 | 128 | 100 |
| 166,1 | 62,9 | 143,2 | 75,3 | 0,379 | 13,5 | 129 | 100 |
| 134,6 | 62,0 | 110,0 | 86,3 | 0,460 | 11,5 | 114 | 110 |
| 171,0 | 76,8 | 136,0 | 114,2 | 0,449 | 14,6 | 118 | 100 |
| 196,9 | 83,5 | 183,0 | 100,5 | 0,424 | 16,5 | 138 | 90 |
| 123,3 | 52,0 | 107,3 | 65,7 | 0,422 | 10,3 | 159 | 90 |
| 127,1 | 52,0 | 123,3 | 58,4 | 0,409 | 10,5 | 144 | 90 |
| 218,4 | 87,7 | | | 0,401 | 18,0 | E | 75 |
| 204,0 | 102,0 | | | 0,500 | 18,0 | E | 100 |

On constate que le contacteur à agitation mécanique permet d'obtenir une teneur résiduelle en méthanol souhaitée (généralement inférieure à 1000 ppm). En revanche, le système présente une tendance à s'engorger (lettre E dans le tableau) lorsque le débit spécifique avoisine 18 m³/m²/h. L'engorgement correspond à une phase organique qui stagne, empêchant tout mouvement dans la colonne et donc toute séparation.

Dans le tableau 2 ci-dessous sont rassemblés les résultats obtenus avec le contacteur à garnissage, dans différentes conditions opérationnelles.

**Tableau 2 - résultats obtenus avec le contacteur à garnissage**

| Débit PO en entrée (kg/h) | Débit PA en entrée (kg/h) | Débit PO en sortie (kg/h) | Débit PA en sortie (kg/h) | Rapport PA / PO à l'entrée | Productivité (m³/m²/h) | Teneur résiduelle méthanol dans PO en sortie (ppm) |
|---|---|---|---|---|---|---|
| 474 | 166 | 400 | 235 | 0,350 | 26,2 | 1134 |
| 565,6 | 185,2 | 473 | 293 | 0,327 | 30,7 | 360 |
| 513 | 179 | 437 | 271 | 0,349 | 28,3 | 604 |
| 328 | 163 | 280 | 195 | 0,497 | 20,0 | 604 |
| 350,4 | 125 | 290 | 183,4 | 0,357 | 19,4 | 1117 |
| 489 | 182,0 | 412 | 266 | 0,372 | 27,4 | 277 |
| 499 | 180,6 | 420 | 270,6 | 0,362 | 27,8 | 824 |
| 499 | 200,4 | 420 | 272,4 | 0,402 | 28,6 | 239 |
| 492 | 147,0 | 418 | 225 | 0,299 | 26,2 | 1091 |
| 239,5 | 83,7 | 204 | 126,6 | 0,349 | 13,2 | 3538 |
| 635 | 220,0 | 522 | 324,4 | 0,346 | 35,0 | 519 |

On constate que le contacteur à garnissage offre également des performances de séparation convenables, tout en permettant un débit spécifique de 1,5 à 2 fois supérieur à celui atteignable avec la colonne à agitation mécanique.

Ce résultat avantageux obtenu avec un contacteur statique peut être également atteint dans d'autres configurations d'extraction liquide-liquide, lorsque la différence de masse volumique et la tension interfaciale entre les deux flux liquides sont faibles.

## Revendications

1. Procédé d'extraction liquide - liquide, comprenant :
- la fourniture d'un flux liquide principal d'entrée, contenant aumoins un composé d'intérêt qui est un ester acrylique, et uncomposé excédentaire qui est un alcool ;
- la fourniture d'un flux liquide de lavage qui est un flux aqueux;
- l'extraction de l'alcool du flux liquide principal d'entrée par contact avec le flux liquide de lavage, permettant de collecterun flux liquide principal de sortie, appauvri en alcool par rapport au flux liquide principal d'entrée ;
**caractérisé en ce que** :
- le flux liquide principal d'entrée et le flux liquide de lavage présentent une différence de masse volumique inférieure ouégale à 50 kg/m³ ainsi qu'une tension interfaciale inférieure ou égale à 3 dyn/cm ; et
- l'étape d'extraction est effectuée dans un contacteur à garnissage ayant une surface spécifique supérieure ou égaleà 200 m²/m³, avec un rapport massique en entrée flux liquidede lavage / flux liquide principal allant de 0,3 à 0,5.

2. Procédé selon la revendication 1, **caractérisé en ce que** l'ester acrylique est l'acrylate de méthyle et/ou l'acrylate d'éthyle, et l'alcool est le méthanol et/ou l'éthanol.

3. Procédé selon la revendication 1 ou 2, **caractérisé en ce que** le flux liquide principal d'entrée contient de 0,5 à 30 % d'alcool, de préférence de 1 à 20 % et plus particulièrement de 2 à 10 % ; et/ou le flux liquide principal de sortie contient moins de 2000 ppm d'alcool, de préférence moins de 1000 ppm, et plus particulièrement moins de 750 ppm voire moins de 500 ppm.

4. Procédé selon l'une des revendications 1 à 3, **caractérisé en ce que** le contacteur à garnissage comporte des éléments de garnissage en vrac, de forme externe essentiellement cylindrique, présentant un diamètre supérieur ou égal au diamètre critique, exprimé en m, d_{c}=2,42×(S*gc/Δρ*g)^{0,5}, S représentant la tensioninterfaciale entre le flux liquide principal d'entrée et le flux liquidede lavage, exprimée en N/m, et Δρ représentant la différence demasse volumique entre le flux liquide principal d'entrée et le flux liquide de lavage, exprimée en kg/m³, gc étant le facteur de conversion de la constante de pesanteur (kg.m/N.s²) et g la constante gravitationnelle (9,83 m/s²) .

5. Procédé de production d'ester acrylique comprenant :
- l'alimentation d'un réacteur en acide acrylique, en catalyseur et en alcool ;
- le soutirage d'un flux d'ester acrylique en sortie du réacteur ;
- l'extraction liquide - liquide du flux d'ester acrylique par un flux aqueux, permettant de collecter un flux d'ester acrylique purifié, l'extraction liquide - liquide étant effectuée selon le procédé de l'une des revendications 1 à 4, dans lequel le fluxliquide principal d'entrée est le flux d'ester acrylique, le flux liquide de lavage est le flux aqueux, le flux liquide principal de sortie est le flux d'ester acrylique purifié, le composé d'intérêt est l'ester acrylique, et le composé excédentaire est l'alcool.

6. Procédé selon la revendication 5, **caractérisé en ce que** le flux d'ester acrylique purifié est soumis en outre à une ou plusieurs étapes de distillation afin d'éliminer des composés organiques supplémentaires.

7. Procédé selon la revendication 5 ou 6, comprenant une étape de décantation en sortie du réacteur, permettant de collecter une
phase aqueuse en plus du flux d'ester acrylique, la phase aqueuse étant distillée pour récupérer d'une part une fraction riche en alcool qui est recyclée vers le réacteur, et d'autre part une fraction riche en eau qui est utilisée en tant que flux liquide de lavage dans l'étape d'extraction liquide-liquide.

8. Procédé selon la revendication 7, comprenant la collecte d'un flux aqueux enrichi en alcool à l'issue de l'étape d'extraction liquide-liquide et la combinaison de celui-ci avec la phase aqueuse issue du réacteur.

## Patentansprüche

1. Verfahren zur flüssig/flüssig-Extraktion, umfassend:
- Bereitstellen eines eingangsseitigen flüssigen Hauptstoffstroms, der mindestens eine Verbindung von Interesse, bei welcher es sich um einen Acrylester handelt, und eine im Überschuss vorliegende Verbindung enthält, bei welcher es sich um einen Alkohol handelt;
- Bereitstellen eines flüssigen Wasch-Stoffstroms, bei welchem es sich um einen wässrigen Stoffstrom handelt;
- Extraktion des Alkohols aus dem eingangsseitigen flüssigen Hauptstoffstrom durch Inkontaktbringen mit dem flüssigen Wasch-Stoffstrom, sodass ein ausgangsseitiger flüssiger Hauptstoffstrom aufgefangen werden kann, der unter Bezugnahme auf den eingangsseitigen flüssigen Hauptstoffstrom an Alkohol abgereichert ist;
**dadurch gekennzeichnet, dass**:
- der eingangsseitige flüssige Hauptstoffstrom und der flüssige Wasch-Stoffstrom einen Dichteunterschied von höchstens 50 kg/m³ sowie eine Grenzflächenspannung von höchstens 3 dyn/cm aufweisen; und
- der Extraktionsschritt in einer Kontaktvorrichtung mit Einbauten durchgeführt wird, die eine spezifische Oberfläche von mindestens 200 m²/m³ hat, mit einem eingangsseitigen Massenverhältnis von flüssigem Wasch-Stoffstrom / flüssigem Hauptstoffstrom im Bereich von 0,3 bis 0,5.

2. Verfahren nach Anspruch 1, **dadurch gekennzeichnet, dass** es sich bei dem Acrylester um Methylacrylat und/oder Ethylacrylat handelt und es sich bei dem Alkohol um Methanol oder Ethanol handelt.

3. Verfahren nach Anspruch 1 oder 2, **dadurch gekennzeichnet, dass** der eingangsseitige flüssige Hauptstoffstrom 0,5 bis 30 % an Alkohol, vorzugsweise 1 bis 20 % und insbesondere 2 bis 10 % enthält; und/oder der ausgangsseitige flüssige Hauptstoffstrom weniger als 2.000 ppm an Alkohol, vorzugsweise weniger als 1.000 ppm und insbesondere weniger als 750 ppm oder sogar weniger als 500 ppm enthält.

4. Verfahren nach einem der Ansprüche 1 bis 3, **dadurch gekennzeichnet, dass** die Kontaktvorrichtung mit Einbauten lose Einbauelemente aufweist, deren äußere Form im Wesentlichen zylindrisch ist, wobei sie einen Durchmesser haben, der größer oder gleich dem kritischen Durchmesser, ausgedrückt in m, ist, für welchen d_{c} = 2,42×(S*gc/Δρ*g)^{0,5} gilt, wobei S für die Grenzflächenspannung zwischen dem eingangsseitigen flüssigen Hauptstoffstrom und dem flüssigen Wasch-Stoffstrom, ausgedrückt in N/m, steht und Δρ für den Dichteunterschied zwischen dem eingangsseitigen flüssigen Hauptstoffstrom und dem flüssigen Wasch-Stoffstrom, ausgedrückt in kg/m³, steht, wobei mit gc der Umwandlungsfaktor der Schwerefeldkonstante (kg.m/N.s²) bezeichnet wird und mit g die Gravitationskonstante (9,83 m/s²) bezeichnet wird.

5. Verfahren zur Herstellung von Acrylester, umfassend:
- Beschicken eines Reaktors mit Acrylsäure, mit Katalysator und mit Alkohol;
- Entnehmen eines Acrylester-Stoffstroms ausgangsseitig des Reaktors;
- flüssig/flüssig-Extrahieren des Acrylester-Stoffstroms mittels eines wässrigen Stoffstroms, sodass ein aufgereinigter Acrylester-Stoffstrom gewonnen werden kann, wobei die flüssig/flüssig-Extraktion gemäß dem Verfahren nach einem der Ansprüche 1 bis 4 durchgeführt wird, wobei es sich bei dem eingangsseitigen flüssigen Hauptstoffstrom um den Acrylester-Stoffstrom handelt, es sich bei dem flüssigen Wasch-Stoffstrom um den wässrigen Stoffstrom handelt, es sich bei dem ausgangsseitigen flüssigen Hauptstoffstrom um den aufgereinigten Acrylester-Stoffstrom handelt, es sich bei der Verbindung von Interesse um den Acrylester handelt und es sich bei der im Überschuss vorliegenden Verbindung um den Alkohol handelt.

6. Verfahren nach Anspruch 5, **dadurch gekennzeichnet, dass** der aufgereinigte Acrylester-Stoffstrom darüber hinaus einem oder mehreren Destillationsschritten unterzogen wird, um zusätzliche organische Verbindungen zu entfernen.

7. Verfahren nach Anspruch 5 oder 6, wobei es ausgangsseitig des Reaktors einen Schritt des Dekantierens umfasst, welcher es ermöglicht, zusätzlich zu dem Acrylester-Stoffstrom eine wässrige Phase zu aufzufangen, wobei die wässrige Phase destilliert wird, um einerseits eine alkoholreiche Phase, welche zum Reaktor zurückgeführt wird, und andererseits eine Fraktion zu gewinnen, welche als flüssiger Wasch-Stoffstrom im Schritt der flüssig/flüssig-Extraktion verwendet wird.

8. Verfahren nach Anspruch 7, wobei es das Auffangen eines an Alkohol angereicherten Stoffstroms nach Abschluss des Schrittes der flüssig/flüssig-Extraktion sowie das Vereinigen desselben mit der wässrigen Phase umfasst, welche aus dem Reaktor stammt.

## Claims

1. Liquid-liquid extraction process, comprising:
- the provision of a main input liquid stream, comprising at least one compound of interest and an excess compound;
- the provision of a liquid scrubbing stream;
- the extraction of the excess compound from the main input liquid stream by contact with the liquid scrubbing stream, making it possible to collect a main output liquid stream, depleted in excess compound with respect to the main input liquid stream;
in which:
- the main input liquid stream and the liquid scrubbing stream exhibit a difference in density of less than or equal to 50 kg/m³ and also an interfacial tension of less than or equal to 3 dyn/cm; and
- the extraction stage is carried out in a packed contactor with a ratio by weight as input liquid scrubbing stream / main liquid stream ranging from 0.3 to 0.5.

2. Process according to Claim 1, in which:
- the main input liquid stream is an organic stream and the liquid scrubbing stream is an aqueous stream;
- preferably, the compound of interest is an acrylic ester, more particularly preferably methyl acrylate and/or ethyl acrylate, and the excess compound is an alcohol, preferably methanol and/or ethanol.

3. Process according to Claim 1 or 2, in which the main input liquid stream comprises from 0.5% to 30% of excess compound, preferably from 1% to 20% and more particularly from 2% to 10%, and/or in which the main output liquid stream comprises less than 2000 ppm of excess compound, preferably less than 1000 ppm and more particularly less than 750 ppm, indeed even less than 500 ppm.

4. Process according to one of Claims 1 to 3, in which the packed contactor comprises packing components exhibiting a diameter of greater than or equal to the critical diameter, expressed in m, resulting from the relationship d_{c}=2.42(S*gc/Δρ*g)^{0.5}, S representing the interfacial tension between the main input liquid stream and the liquid scrubbing stream, expressed in N/m, Δρ representing the difference in density between the main input liquid stream and the liquid scrubbing stream, expressed in kg/m³, gc being the conversion factor for the gravitational constant (kg.m/N.s²) and g being the gravitational constant (9.83 m/s²).

5. Process for the production of acrylic ester comprising:
- the feeding of a reactor with acrylic acid, with catalyst and with alcohol;
- the withdrawal of an acrylic ester stream at the outlet of the reactor;
- the liquid-liquid extraction of the acrylic ester stream by an aqueous stream, which makes it possible to collect a purified acrylic ester stream, the liquid-liquid extraction being carried out according to the process of one of Claims 1 to 4, in which the main input liquid stream is the acrylic ester stream, the liquid scrubbing stream is the aqueous stream, the main output liquid stream is the purified acrylic ester stream, the compound of interest is the acrylic ester and the excess compound is the alcohol.

6. Process according to Claim 5, in which the purified acrylic ester stream is additionally subjected to one or more distillation stages in order to remove additional organic compounds.

7. Process according to Claim 5 or 6, comprising a settling stage at the outlet of the reactor, making it possible to collect an aqueous phase in addition to the acrylic ester stream, the aqueous phase being distilled in order to recover, on the one hand, a fraction rich in alcohol which is recycled to the reactor and, on the other hand, a fraction rich in water which is used as liquid scrubbing stream in the liquid-liquid extraction stage.

8. Process according to Claim 7, comprising the collecting of an aqueous stream enriched in alcohol on conclusion of the liquid-liquid extraction stage and the combining of this with the aqueous phase resulting from the reactor.
